# EUROPÄISCHE PATENTSCHRIFT

(11) **EP 3 817 575 B1**
(45) Veröffentlichungstag und Bekanntmachung des Hinweises auf die Patenterteilung: **15.10.2025**
(21) Anmeldenummer: 18742424.7
(22) Anmeldetag: 06.07.2018
(51) Int. Cl.: A61K 8/02, A61K 8/34, A61K 8/42, A61Q 19/00, A61Q 11/00, A23G 4/06, A23L 29/30, A23L 27/00, A23L 27/20, A23L 27/30, A23L 33/125

(54) **SOLUBILISIERUNG SCHWERLÖSLICHER KÜHLSUBSTANZEN**
SOLUBILISATION OF COOLING AGENTS OF LOW SOLUBILITY
SOLUBILISATION D`AGENT DE REFROIDISSEMENT DE FAIBLE SOLUBILITÉ

(43) Veröffentlichungstag der Anmeldung: 12.05.2021
(73) Patentinhaber: Symrise AG, 37603 Holzminden Niedersachsen (DE)
(72) Erfinder: WEISSBRODT, Jenny, 37603 Holzminden (DE); BACKES, Michael, 37603 Holzminden (DE); LAGES, Rita, 37619 Bodenwerder (DE); PAULI, Olga, 37603 Holzminden (DE)
(74) Vertreter: Eisenführ Speiser
(86) Internationale Anmeldenummer: PCT/EP2018/068365
(87) Internationale Veröffentlichungsnummer: WO 2020/007484

(56) Entgegenhaltungen:
- EP-A1- 2 186 506
- EP-B1- 2 186 506
- WO-A1-2017/165882
- WO-A1-2018/028770
- CN-A- 107 890 074
- DATABASE WPI Week 201832, Derwent World Patents Index; AN 2018-28934D

## Beschreibung

Die vorliegende Erfindung betrifft eine kühlende Zusammensetzung zur Herstellung von kühlenden Partikeln umfassend eine Lösungsmittelkomponente und darin gelöst (1R,2S,5R)-N-(4-Methoxyphenyl)-5-methyl-2-(1-methylethyl)-cyclohexan-carboxamid. Die vorliegende Erfindung betrifft zudem kühlende Partikel umfassend eine erfindungsgemäße, kühlende Zusammensetzung. Weiterhin betrifft die vorliegende Erfindung orale Zubereitungen, umfassend kühlende Partikel, sowie eine Verwendung von kühlenden Partikeln. Schließlich stellt die vorliegende Erfindung auch ein Verfahren zur Herstellung von kühlenden Partikeln bereit und es wird eine Verwendung einer Lösungsmittelkomponente zum Lösen von Kühlwirkstoffen beschrieben.

Aus dem Stand der Technik sind Verfahren zur Herstellung von kühlenden Partikeln beschrieben. In der EP 2 801 263 A1 wird eine kühlende Zusammensetzung in einem Sprühgranulationsprozess verkapselt. Die in dem Verfahren eingesetzte kühlende Zusammensetzung umfasst einen Kühlwirkstoff und zumindest eine hydrophobe Verbindung sowie optional einen Alkohol. Vor dem Sprühgranulationsprozess werden die Komponenten der kühlenden Zusammensetzung bei erhöhter Temperatur von bis zu 100°C gelöst und bis zur weiteren Verarbeitung bei erhöhten Temperaturen von 35°C bis 65°C gehalten, um eine Rekristallisation der Komponenten, insbesondere des Kühlwirkstoffes, zu verhindern.

Das im Stand der Technik beschriebene Verfahren hat den Nachteil, dass die eingesetzte kühlende Zusammensetzung nicht lagerstabil ist und bis zu ihrer Verwendung bei erhöhter Temperatur gehalten werden muss. Um die Zusammensetzung bei dieser erhöhten Temperatur zu halten, bedarf es eine spezielle Anlage, die ein Warmhalten der verwendeten Edukte ermöglicht. Zum anderen wird bei dem Erwärmen und Warmhalten der Zusammensetzung sehr viel Energie verbraucht, was zu einer Erhöhung der Produktionskosten führt.

Ebenfalls nachteilig bei dem im Stand der Technik beschriebenen Verfahren ist, dass durch das nötige Warmhalten der gelösten Zusammensetzung ein Transport der hergestellten Zusammensetzung nicht oder nur unter enormen Aufwand möglich ist, sodass der Lösungsprozess und die anschließende Verwendung der Zusammensetzung am gleichen Ort erfolgen muss. Ein Abfüllen der gelösten Zusammensetzung in Fässer mit anschließendem Transport oder einer Lagerung bei normalen Außentemperaturen, ggf. sogar bei tiefen Temperaturen im Winter, ist mit den im Stand der Technik beschriebenen Zusammensetzungen bisher nicht möglich.

Da eine Lagerung bzw. ein Transport der Zusammensetzung bisher nicht ohne permanente Erwärmung möglich ist und das Lösen bzw. Herstellen der Zusammensetzung somit erst am Ort der Weiterverarbeitung erfolgen musste, ist es bei den im Stand der Technik beschriebenen Zusammensetzung nötig, die einzelnen Bestandteile der Zusammensetzung in verschiedene Komponenten aufzuteilen, die erst vor dem Auflösen und somit dem Herstellen der Zusammensetzung in der Hitze gemischt wurden.

WO 2018/028770 A1 beschreibt Lösungsmittel für (E)-3-Benzo[I,3]Dioxol-5-yl-N,N-Diphenyl-2-Propenamid, unter anderem auch 2-Hydroxypropyl (2-isopropyl-5-methylcyclohexyl) carbonat. Jedoch wird kein Hinweis auf mögliche weitere Verwendungen von 2-Hydroxypropyl (2-isopropyl-5-methylcyclohexyl) carbonat gegeben.

EP 2 186 506 A1 beschreibt eine Zusammensetzung enthaltend WS-12, das in der Zusammensetzung gelöst ist, sowie Menthol.

WO 2017/165882 A1 beschreibt wasserunlösliche Kühlwirkstoffe, die in einer wasserlöslichen Matrix verkapselt wurden.

CN 107890074 A beschreibt eine Kühlwirkstoff-Zusammensetzung, die N-ethyl-p-menthyl-3-carboxamid (WS-3) enthält, und deren Herstellungsverfahren.

Aufgabe der vorliegenden Erfindung war es, kühlende Zusammensetzungen bereit zu stellen, die die oben genannten Nachteile von bereits bekannten kühlenden Zusammensetzungen nicht aufweisen. Insbesondere war es Aufgabe der vorliegenden Erfindung kühlende Zusammensetzungen bereit zu stellen, die zumindest eine der nachfolgenden Anforderungen erfüllen, nämlich
- bei üblichen Temperaturen in einer Produktionshalle im Bereich von 15 bis 30°C ohne zusätzliche Erwärmung der Zusammensetzung gelagert werden können
   und
- ohne besondere Vorkehrungen transportiert werden können, ohne dass einzelne Bestandteile der Zusammensetzung auskristallisieren

Die Aufgabe wird erfindungsgemäß gelöst durch eine kühlende Zusammensetzung zur Herstellung von kühlenden Partikeln bestehend aus oder umfassend
(A) eine Lösungsmittelkomponente bestehend aus oder umfassend
   i) 2-Hydroxypropyl (2-isopropyl-5-methyl-cyclohexyl) carbonat
      und/oder
   ii) (2-Isopropyl-5-methyl-cyclohexyl) 2-hydroxypropanoat
   und darin gelöst
(B) 0,5 bis 12 Gew.-% (1R,2S,5R)-*N*-(4-Methoxyphenyl)-5-methyl-2-(1-methylethyl)-cyclohexan-carboxamid (CAS Nummer 68489-09-8; FEMA Nummer 4681) als Kühlwirkstoff, bezogen auf das Gesamtgewicht der Zusammensetzung.

Es hat sich überraschenderweise gezeigt, dass der Kühlwirkstoff (1R,2S,5R)-N-(4-Methoxyphenyl)-5-methyl-2-(1-methylethyl)-cyclohexan-carboxamid in 2-Hydroxypropyl (2-isopropyl-5-methyl-cyclohexyl) carbonat, (2-Isopropyl-5-methyl-cyclohexyl) 2-hydroxypropanoat oder einer Mischung aus beiden Verbindungen gelöst werden kann und dass die resultierende Lösung auch bei Temperaturen von 25°C und weniger über Tage und Wochen lagerstabil sind. Dies ist insbesondere überraschend, da es sich bei (2-Isopropyl-5-methyl-cyclohexyl) 2-hydroxypropanoat um einen Feststoff mit einem Schmelzpunkt von ca. 40°C handelt. Dabei können Mengen von bis zu 12 Gew.-% des Kühlwirkstoffes, bezogen auf das Gesamtgewicht der Zusammensetzung, gelöst werden.

In einer Ausgestaltung der vorliegenden Erfindung enthält die Zusammensetzung zusätzlich N-Ethyl-2-isopropyl-5-methyl-cyclohexanecarboxamid. Eigene Untersuchungen haben gezeigt, dass N-Ethyl-2-isopropyl-5-methyl-cyclohexanecarboxamid, das ebenfalls ein Feststoff ist, zwar nicht in der Lage ist, den Kühlwirkstoff zu Lösen bzw. die durch Erhitzen gewonnen Schmelzen aus N-Ethyl-2-isopropyl-5-methyl-cyclohexanecarboxamid und dem Kühlwirkstoff beim Abkühlen auskristallisieren. Allerdings kann der Zusatz von N-Ethyl-2-isopropyl-5-methyl-cyclohexanecarboxamid die erfindungsgemäßen Zusammensetzungen stabilisieren, sodass kein Auskristallisieren erfolgt. Dies ist in der Hinsicht besonders überraschend, da der Fachmann erwarten würde, dass bei der Zugabe von weiteren Feststoffen die maximale Löslichkeit von anderen Feststoffen erniedrigt wird und somit eher ein Auskristallisieren von bereits gelösten Feststoffen begünstigt wird. Dies dem Fachmann bekannte Effekt wird beispielsweise beim Aussalzen ausgenutzt, bei dem wasserlösliche Substanzen durch eine Salzzugabe aus der wässrigen Phase verdrängt werden.

In der vorliegenden Erfindung wird das N-Ethyl-2-isopropyl-5-methyl-cyclohexanecarboxamid der Gruppe (A), d.h. der Lösungsmittelkomponente, zugeordnet auch wenn diese Verbindung selbst nicht geeignet ist, um (1R,2S,5R)-N-(4-Methoxyphenyl)-5-methyl-2-(1-methylethyl)-cyclohexan-carboxamid ohne die Zugabe von weiteren Verbindungen über lange Zeit zu lösen. Es dient im vorliegenden Fall als Lösungs-Stabilisator und verbessert das Lösungsvermögen der gesamten Lösungsmittelkomponenten bezüglich der Löslichkeit von (1R,2S,5R)-N-(4-Methoxyphenyl)-5-methyl-2-(1-methylethyl)-cyclohexan-carboxamid.

Erfindungsgemäß besonders bevorzugt sind somit kühlende Zusammensetzung zur Herstellung von kühlenden Partikeln, die
(A) eine Lösungsmittelkomponente bestehend aus oder umfassend
   i) 2-Hydroxypropyl (2-isopropyl-5-methyl-cyclohexyl) carbonat
      und
   ii) (2-Isopropyl-5-methyl-cyclohexyl) 2-hydroxypropanoat
      und
   iii) N-Ethyl-2-isopropyl-5-methyl-cyclohexanecarboxamid
   und darin gelöst
(B) 0,5 bis 12 Gew.-% (1R,2S,5R)-*N*-(4-Methoxyphenyl)-5-methyl-2-(1-methylethyl)-cyclohexan-carboxamid (CAS Nummer 68489-09-8; FEMA Nummer 4681) als Kühlwirkstoff, bezogen auf das Gesamtgewicht der Zusammensetzung.

Eigene Untersuchungen haben völlig überraschend gezeigt, dass die Kombination aus 2-Hydroxypropyl (2-isopropyl-5-methyl-cyclohexyl) carbonat, (2-Isopropyl-5-methyl-cyclohexyl) 2-hydroxypropanoat und N-Ethyl-2-isopropyl-5-methyl-cyclohexanecarboxamid besonders gut geeignet ist, um (1R,2S,5R)-*N*-(4-Methoxyphenyl)-5-methyl-2-(1-methylethyl)-cyclohexan-carboxamid zu Lösen und dass die resultierenden Lösungen besonders lange bei Temperaturen von 25°C und geringer stabil bleiben ohne dass einzelne Komponenten auskristallisieren.

Kühlende Zusammensetzungen sind erfindungsgemäß bevorzugt, wobei es sich bei N-Ethyl-2-isopropyl-5-methyl-cyclohexanecarboxamid um (1R,2S,5R)-N-Ethyl-2-isopropyl-5-methyl-cyclohexanecarboxamid (CAS Nummer 68489-00-9) handelt.

Kühlende Zusammensetzungen sind erfindungsgemäß bevorzugt, wobei es sich bei 2-Hydroxypropyl (2-isopropyl-5-methyl-cyclohexyl) carbonat um 2-Hydroxypropyl [(1R,2S,5R)-2-isopropyl-5-methyl-cyclohexyl] carbonat (CAS Nummer 260781-16-6) handelt.

Eigene Untersuchungen haben überraschenderweise gezeigt, dass das hier beschriebenen Diastereomer der erfindungsgemäß verwendeten Verbindung gute Eigenschaften bezüglich der Löslichkeit von (1R,2S,5R)-*N*-(4-Methoxyphenyl)-5-methyl-2-(1-methylethyl)-cyclohexan-carboxamid aufweisen.

Kühlende Zusammensetzungen sind erfindungsgemäß bevorzugt, wobei es sich bei (2-Isopropyl-5-methyl-cyclohexyl) 2-hydroxypropanoat um [(1R,2S,5R)-2-Isopropyl-5-methylcyclohexyl] (2S)-2-hydroxypropanoat (CAS Nummer 61597-98-6) handelt.

Eigene Untersuchungen haben überraschenderweise gezeigt, dass das hier beschriebenen Diastereomer der erfindungsgemäß verwendeten Verbindung gute Eigenschaften bezüglich der Löslichkeit von (1R,2S,5R)-*N*-(4-Methoxyphenyl)-5-methyl-2-(1-methylethyl)-cyclohexan-carboxamid aufweisen.

Dabei ist es erfindungsgemäß besonders bevorzugt, wenn es sich bei dem eingesetzten N-Ethyl-2-isopropyl-5-methyl-cyclohexanecarboxamid um (1R,2S,5R)-N-Ethyl-2-isopropyl-5-methyl-cyclohexanecarboxamid (CAS Nummer 68489-00-9) handelt, es sich bei dem eingesetzten 2-Hydroxypropyl (2-isopropyl-5-methyl-cyclohexyl) carbonat um 2-Hydroxypropyl [(1R,2S,5R)-2-isopropyl-5-methyl-cyclohexyl] carbonat (CAS Nummer 260781-16-6) handelt und es sich bei dem eingesetzten (2-Isopropyl-5-methyl-cyclohexyl) 2-hydroxypropanoat um [(1R,2S,5R)-2-Isopropyl-5-methyl-cyclohexyl] (2S)-2-hydroxypropanoat (CAS Nummer 61597-98-6) handelt.

Eigene Untersuchungen haben überraschenderweise gezeigt, dass die Kombination der drei beschriebenen Diastereomere der erfindungsgemäß verwendeten Verbindung zu Hervorragenden Eigenschaften bezüglich der Löslichkeit von (1R,2S,5R)-N-(4-Methoxyphenyl)-5-methyl-2-(1-methylethyl)-cyclohexan-carboxamid führen.

Die oben beschriebenen Diastereomere der Lösungsmittelkomponenten haben sich als besonders vorteilhaft erwiesen, da sie nicht nur zu einer ausgezeichneten Solubilisierung des (1R,2S,5R)-*N*-(4-Methoxyphenyl)-5-methyl-2-(1-methylethyl)-cyclohexan-carboxamids führen, sondern zusätzlich auch eine kühlende Wirkung aufweisen und somit die kühlende Wirkung der erfindungsgemäßen Zusammensetzung noch weiter verbessern.

Kühlende Zusammensetzungen sind erfindungsgemäß bevorzugt, wobei die Lösungsmittelkomponente
2 bis 20 Gew.-%, vorzugsweise 4 bis 15 Gew.-%, besonders bevorzugt 6,4 bis 11,8 Gew.-% 2-Hydroxypropyl (2-isopropyl-5-methyl-cyclohexyl) carbonat umfasst,
   und/oder
55 bis 75 Gew.-%, vorzugsweise 60 bis 70 Gew.-%, besonders bevorzugt 63 bis 67 Gew.-% (2-Isopropyl-5-methyl-cyclohexyl) 2-hydroxypropanoat umfasst,
   und/oder
15 bis 35 Gew.-%, vorzugsweise 20 bis 30 Gew.-%, besonders bevorzugt 23 bis 27 Gew.-% N-Ethyl-2-isopropyl-5-methyl-cyclohexanecarboxamid umfasst,
jeweils bezogen auf das Gesamtgewicht der Lösungsmittelkomponente.

Eine erfindungsgemäße kühlende Zusammensetzung ist bevorzugt, wobei die Zusammensetzung 88 bis 99,5 Gew.-%, vorzugsweise 90 bis 94 Gew.-% der Lösungsmittelkomponente (A) enthält, bezogen auf das Gesamtgewicht der Zusammensetzung.

Eine erfindungsgemäße kühlende Zusammensetzung ist bevorzugt, wobei die Zusammensetzung zusätzlich 0,5 bis 8 Gew.-%, vorzugsweise 4 bis 7,5, besonders bevorzugt 5 bis 7 Gew.-% mittelkettigen Triglyceride enthält, bezogen auf das Gesamtgewicht der Zusammensetzung.

Eigene Untersuchungen haben überraschenderweise gezeigt, dass die Zugabe von mittelkettigen Triglyceriden die Stabilität von (1R,2S,5R)-*N*-(4-Methoxyphenyl)-5-methyl-2-(1-methylethyl)-cyclohexan-carboxamid in Lösung noch weiter verbessern kann. Dieses Ergebnis ist in der Hinsicht überraschend, da mittelkettige Triglyceride für (1R,2S,5R)-N-(4-Methoxyphenyl)-5-methyl-2-(1-methylethyl)-cyclohexan-carboxamid als schlechtes Lösungsmittel gelten. Bei der Zugabe eines schlechten Lösungsmittels würde der Fachmann erwarten, dass der gelöste Stoff aus der Lösung auskristallisiert. Dies ist im vorliegenden Fall allerdings überraschenderweise nicht der Fall. Bis zu einem Anteil von 8 Gew.-% stabilisiert das mittelkettige Triglyceride die Lösung.

Mittelkettige Triglyceride (MKT), engl. medium-chain triglycerides (MCTs) oder MCT-Fette, sind Triglyceride, die mittelkettige Fettsäuren enthalten. Zu den mittelkettigen Fettsäuren zählen die Capron- (C 6:0), Capryl- (C 8:0), Caprin- (C 10:0) und die Laurinsäure (C 12:0). Es handelt sich dabei um gesättigte Fettsäuren, welche vor allem in tropischen Pflanzenfetten wie Kokosfett (ca. 60 %) und Palmkernöl (ca. 55 %) und in Butter vorkommen. Zu einem geringen Teil sind sie auch im Milchfett (ca. 10 %) enthalten. Mittelkettige Triglyceride werden industriell durch Hydrolyse von Kokosfett und Palmkernöl, Fraktionierung der mittelkettigen Fettsäuren und anschließender Veresterung mit Glycerin gewonnen. Ein reines mittelkettiges Triglycerid ist farblos bis gelblich, neutral in Geruch und Geschmack und von sehr niedriger Viskosität.

Erfindungsgemäß bevorzugt weist ein mittelkettiges Triglycerid 50-80 % Caprylsäure C8, 25-45 % Caprinsäure C10, Laurinsäure max. 3 % und max. 2 % Capronsäure auf.

Eine erfindungsgemäße kühlende Zusammensetzung ist bevorzugt, wobei die Zusammensetzung mehr als 50 Gew.-%, vorzugsweise mehr als 65 Gew.-%, besonders bevorzugt mehr als 80 Gew.-% der Lösungsmittelkomponente (A) enthält, bezogen auf das Gesamtgewicht der Zusammensetzung.

Eine erfindungsgemäße kühlende Zusammensetzung ist besonders bevorzugt, wobei die Zusammensetzung 80 bis 99 Gew.-%, vorzugsweise 80,5 bis 95,5 Gew.-%, besonders bevorzugt 81 bis 94,5 Gew.-% der Lösungsmittelkomponente (A) enthält, bezogen auf das Gesamtgewicht der Zusammensetzung.

Eine erfindungsgemäße kühlende Zusammensetzung ist besonders bevorzugt, wobei die Zusammensetzung 0,5 bis 6 Gew.-%, vorzugsweise 1 bis 4 Gew.-%, besonders bevorzugt 1,5 bis 3 Gew.-% (1R,2S,5R)-*N*-(4-methoxyphenyl)-5-methyl-2-(1-methylethyl)-cyclohexane-carboxamid (FEMA 4681) als Kühlwirkstoff umfasst, jeweils bezogen auf das Gesamtgewicht der Zusammensetzung.

Erfindungsgemäße kühlende Zusammensetzung mit einem Anteil von 0,5 bis 6 Gew.-% Kühlwirkstoff sind besonders bevorzugt, wenn die kühlende Zusammensetzung direkt in flüssigen Halbfertigprodukten oder Fertigprodukten verwendet werden soll.

Eine erfindungsgemäße kühlende Zusammensetzung ist besonders bevorzugt, wobei die Zusammensetzung 6 bis 12 Gew.-%, vorzugsweise 6,5 bis 10 Gew.-%, besonders bevorzugt 7 bis 9 Gew.-% (1R,2S,5R)-*N*-(4-methoxyphenyl)-5-methyl-2-(1-methylethyl)-cyclohexane-carboxamid (FEMA 4681) als Kühlwirkstoff umfasst, jeweils bezogen auf das Gesamtgewicht der Zusammensetzung.

Erfindungsgemäße kühlende Zusammensetzung mit einem Anteil von 6 bis 12 Gew.-% Kühlwirkstoff sind besonders bevorzugt, wenn die kühlende Zusammensetzung für die Herstellung von kühlenden Partikeln oder in festen Halbfertigprodukten oder Fertigprodukten verwendet werden soll.

Erfindungsgemäß besonders bevorzugt sind somit kühlende Zusammensetzung zur Herstellung von kühlenden Partikeln, die
(A) eine Lösungsmittelkomponente bestehend aus oder umfassend
   i) 2 bis 20 Gew.-%, vorzugsweise 4 bis 15 Gew.-%, besonders bevorzugt 6,4 bis 11,8 Gew.-% 2-Hydroxypropyl [(1R,2S,5R)-2-isopropyl-5-methyl-cyclohexyl] carbonat (CAS Nummer 260781-16-6)
      und
   ii) 55 bis 75 Gew.-%, vorzugsweise 60 bis 70 Gew.-%, besonders bevorzugt 63 bis 67 Gew.-% [(1R,2S,5R)-2-Isopropyl-5-methyl-cyclohexyl] (2S)-2-hydroxypropanoat (CAS Nummer 61597-98-6) umfasst
      und
   iii) 15 bis 35 Gew.-%, vorzugsweise 20 bis 30 Gew.-%, besonders bevorzugt 23 bis 27 Gew.-% (1R,2S,5R)-*N*-Ethyl-2-isopropyl-5-methyl-cyclohexanecarboxamid (CAS Nummer 68489-00-9) umfasst,
   jeweils bezogen auf das Gesamtgewicht der Lösungsmittelkomponente
   und darin gelöst
(B) 0,5 bis 12 Gew.-% (1R,2S,5R)-*N*-(4-Methoxyphenyl)-5-methyl-2-(1-methylethyl)-cyclohexan-carboxamid (CAS Nummer 68489-09-8; FEMA Nummer 4681) als Kühlwirkstoff, bezogen auf das Gesamtgewicht der Zusammensetzung,
und wobei die Zusammensetzung mehr als 50 Gew.-%, vorzugsweise mehr als 65 Gew.-%, besonders bevorzugt mehr als 80 Gew.-% der Lösungsmittelkomponente (A) enthält, bezogen auf das Gesamtgewicht der Zusammensetzung.

Erfindungsgemäß ebenfalls besonders bevorzugt sind somit kühlende Zusammensetzung zur Herstellung von kühlenden Partikeln, die
(A) eine Lösungsmittelkomponente bestehend aus oder umfassend
   i) 2 bis 20 Gew.-%, vorzugsweise 4 bis 15 Gew.-%, besonders bevorzugt 6,4 bis 11,8 Gew.-% 2-Hydroxypropyl [(1R,2S,5R)-2-isopropyl-5-methyl-cyclohexyl] carbonat (CAS Nummer 260781-16-6)
      und
   ii) 55 bis 75 Gew.-%, vorzugsweise 60 bis 70 Gew.-%, besonders bevorzugt 63 bis 67 Gew.-% [(1R,2S,5R)-2-Isopropyl-5-methyl-cyclohexyl] (2S)-2-hydroxypropanoat (CAS Nummer 61597-98-6) umfasst
      und
   iii) 15 bis 35 Gew.-%, vorzugsweise 20 bis 30 Gew.-%, besonders bevorzugt 23 bis 27 Gew.-% (1R,2S,5R)-*N*-Ethyl-2-isopropyl-5-methyl-cyclohexanecarboxamid (CAS Nummer 68489-00-9) umfasst,
   jeweils bezogen auf das Gesamtgewicht der Lösungsmittelkomponente
   und darin gelöst
(B) 0,5 bis 12 Gew.-% (1R,2S,5R)-*N*-(4-Methoxyphenyl)-5-methyl-2-(1-methylethyl)-cyclohexan-carboxamid (CAS Nummer 68489-09-8; FEMA Nummer 4681) als Kühlwirkstoff, bezogen auf das Gesamtgewicht der Zusammensetzung,

und wobei die Zusammensetzung zusätzlich 0,5 bis 8 Gew.-%, vorzugsweise 4 bis 7,5, besonders bevorzugt 5 bis 7 Gew.-% mittelkettige Triglyceride enthält, bezogen auf das Gesamtgewicht der Zusammensetzung,
wobei die Zusammensetzung mehr als 50 Gew.-%, vorzugsweise mehr als 65 Gew.-%, besonders bevorzugt mehr als 80 Gew.-% der Lösungsmittelkomponente (A) enthält, bezogen auf das Gesamtgewicht der Zusammensetzung.

Auch wenn der Zusatz von weiteren Lösungsmitteln, wie beispielsweise Alkoholen, zu den erfindungsgemäßen kühlenden Zusammensetzungen nicht ausgeschlossen ist, so ist dies für die Solubilisierung des (1R,2S,5R)-*N*-(4-Methoxyphenyl)-5-methyl-2-(1-methylethyl)-cyclohexan-carboxamids nicht nötig. Es ist erfindungsgemäß bevorzugt, wenn die kühlenden Zusammensetzungen keine Alkohole, wie beispielsweise wie Methanol, Ethanol, Propanol, 1,2-Propandiol, enthalten.

Ein weiterer Aspekt der vorliegenden Erfindung betrifft kühlende Partikel umfassend eine erfindungsgemäße kühlende Zusammensetzung.

Es hat sich in eigenen Untersuchungen gezeigt, dass erfindungsgemäße kühlende Partikel ein stärkeres Frischegefühl durch eine physiologisch kühlende Wirkung des Kühlwirkstoffes vermitteln, als kühlende Partikel, die die gleiche Menge Kühlwirkstoff aufweisen allerdings eine andere Zusammensetzung der Lösungsmittelkomponente. Durch die sehr gute Löslichkeit des Kühlwirkstoffes in der erfindungsgemäß verwendeten Lösungsmittelkomponente kristallisiert der Kühlwirkstoff nicht in dem Partikel aus. Bei der oralen Aufnahme der kühlenden Partikel liegt der Kühlwirkstoff daher bereits gelöst vor und kann sich daher im Mund wesentlich besser verteilen, als wenn er als kristalliner Festkörper vorliegen würde. Entsprechend führt dies zu einem stärkeren Frischegefühl.

Dieser Effekt wird allerdings nicht nur bei erfindungsgemäßen kühlenden Partikeln beobachtet, sondern auch bei der oralen Aufnahme von erfindungsgemäßen Zusammensetzungen allgemein.

Bei den erfindungsgemäßen kühlenden Partikeln kann es sich vorzugsweise um Matrixpartikel oder Granulate handeln, die beispielsweise mittels Sprühtrocknung oder mittels Sprühgranulation gewonnen wurden, oder um Kern-Hülle Partikel.

Erfindungsgemäß bevorzugt sind kühlende Partikel mit einem mittleren Partikeldurchmesser D50 im Bereich von 10 bis 2000 µm.

Sofern es sich bei den erfindungsgemäßen Partikeln um Matrixpartikel handelt, die mittels Sprühtrocknung gewonnen wurden, ist der mittlere Partikeldurchmesser D50 vorzugsweise im Bereich von 10 bis 200 µm, besonders bevorzugt im Bereich von 15 bis 150 µm, ganz besonders bevorzugt im Bereich von 50 bis 100 µm.

Sofern es sich bei den erfindungsgemäßen Partikeln um Granulate handelt, die mittels Sprühgranulation gewonnen wurden, ist der mittlere Partikeldurchmesser D50 vorzugsweise im Bereich von 200 bis 2000 µm, besonders bevorzugt im Bereich von 250 bis 1500 µm, ganz besonders bevorzugt im Bereich von 300 bis 900 µm.

Der mittlere Partikeldurchmesser D50 wird dabei mittels Laserlichtbeugung nach ISO13320:2009-10 bestimmt.

Erfindungsgemäße kühlende Partikel sind bevorzugt, wobei die Partikel als Matrixmaterialien oder Hüllenmaterialien Verbindungen enthalten oder daraus bestehen ausgewählt aus der Gruppe bestehend aus Stärke, Stärkederivate (z.B. modifizierte Stärke), Cellulose oder Cellulosederivate (z.B. Hydroxypropylcellulose), anderen Polysaccharide (z.B. Pektin, Dextrin, Alginat, Curdlan, Carageenan, Chitin, Chitosan, Pullulan, Gummi arabicum), natürliche Fette, natürliche Wachse (z.B. Bienenwachs, Carnaubawachs), Proteine, z.B. Gelatine und sonstigen Naturprodukte (z.B. Schellack) handelt.

Erfindungsgemäße kühlende Partikel sind bevorzugt, wobei die kühlenden Partikel 5 bis 40 Gew.-%, vorzugsweise 10 bis 30 Gew.-% der erfindungsgemäßen kühlenden Zusammensetzung umfassen, bezogen auf das Gesamtgewicht der Partikel.

Ein weiterer Aspekt der vorliegenden Erfindung betrifft eine orale Zubereitung, umfassend erfindungsgemäße kühlende Partikel oder eine erfindungsgemäße Zusammensetzung.

Eine erfindungsgemäße orale Zubereitung ist bevorzugt, wobei die orale Zubereitung ein Getränk, ein Kaugummi, ein Mundwasser, Zahnpasta, Lippenbalsam oder ein Bonbon ist.

Eine erfindungsgemäße orale Zubereitung ist bevorzugt, umfassend a) 5 bis 95 Gew.-% Kaugummibasis, b) 5 bis 95 Gew.-% Füll- und Süßungsmittel, c) 0,1 bis 15 Gew.-% Geschmackstoffe, d) 0,2 bis 4 Gew.-% erfindungsgemäße kühlende Partikel.

Ein weiterer Aspekt der vorliegenden Erfindung betrifft eine Verwendung von erfindungsgemäßen kühlenden Partikeln oder einer erfindungsgemäßen kühlenden Zusammensetzung, um in oralen Zubereitungen ein Frischegefühl durch eine physiologisch kühlende Wirkung zu vermitteln.

Ein weiterer Aspekt der vorliegenden Erfindung betrifft eine Verwendung einer erfindungsgemäßen Zusammensetzung in einem Verkapselungsverfahren, vorzugsweise einer Sprühtrocknung oder Sprühgranulation.

Ein weiterer Aspekt der vorliegenden Erfindung betrifft ein Verfahren zur Herstellung von kühlenden Partikeln umfassend die folgenden Schritte:
- Herstellen oder Bereitstellen einer erfindungsgemäßen Zusammensetzung,
- Lagern der hergestellten oder bereitgestellten Zusammensetzung,
- Herstellen oder Bereitstellen von Matrixmaterialien,
- gegebenenfalls Mischen der hergestellten oder bereitgestellten Zusammensetzung mit den hergestellten oder bereitgestellten Matrixmaterialien,
- Durchführen eines Verkapselungsverfahrens, sodass kühlende Partikel resultieren, in denen die Zusammensetzung von den Matrixmaterialien umschlossen wird.

Erfindungsgemäß bevorzugt ist ein Verfahren, wobei es sich bei dem Verkapselungsverfahren um Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren handelt, vorzugsweise um Sprühtrocknung oder Sprühgranulation handelt.

Ein erfindungsgemäßes Verfahren ist bevorzugt, wobei es sich bei den Matrixmaterialien um Stärke, Stärkederivate (z.B. modifizierte Stärke), Cellulose oder Cellulosederivate (z.B. Hydroxypropylcellulose), anderen Polysaccharide (z.B. Pektin, Dextrin, Alginat, Curdlan, Carageenan, Chitin, Chitosan, Pullulan, Gummi arabicum), natürliche Fette, natürliche Wachse (z.B. Bienenwachs, Carnaubawachs), Proteine, z.B. Gelatine und/oder sonstigen Naturprodukte (z.B. Schellack) handelt.

Ein erfindungsgemäßes Verfahren ist bevorzugt, wobei das Lagern der hergestellten oder bereitgestellten Zusammensetzung bei einer Temperatur unterhalb von 40°C, vorzugsweise unterhalb von 35°C, besonders bevorzugt unterhalb von 30°C, weiter bevorzugt unterhalb von 25°C erfolgt. In einigen Ausgestaltungen der vorliegenden Erfindung ist es bevorzugt, wenn die Lagerung der hergestellten oder bereitgestellten Zusammensetzung bei einer Temperatur im Bereich von 0°C bis 25°C erfolgt, vorzugsweise im Bereich von 5°C bis 15°C erfolgt.

Ein erfindungsgemäßes Verfahren ist bevorzugt, wobei das Lagern der hergestellten oder bereitgestellten Zusammensetzung bei einer Temperatur zwischen 5°C und 65°C, vorzugsweise zwischen 18°C und 35°C, besonders bevorzugt zwischen 20 und 25°C erfolgt.

Ein erfindungsgemäßes Verfahren ist bevorzugt, wobei das Lagern der hergestellten oder bereitgestellten Zusammensetzung ohne Zuführung von Wärmeenergie erfolgt.

Ein erfindungsgemäßes Verfahren ist bevorzugt, wobei die erhaltenen, kühlenden Partikel einen mittleren Partikeldurchmesser D50 im Bereich von 10 bis 2000 µm aufweisen.

Ein weiterer Aspekt der vorliegenden Erfindung betrifft eine Verwendung einer Lösungsmittelkomponente bestehend aus oder umfassend
i) 2-Hydroxypropyl (2-isopropyl-5-methyl-cyclohexyl) carbonat
   und/oder
ii) (2-Isopropyl-5-methyl-cyclohexyl) 2-hydroxypropanoat
zum Lösen eines oder mehrerer Kühlwirkstoffe.

Eigene Untersuchungen haben überraschenderweise gezeigt, dass die in erfindungsgemäßen Zusammensetzung verwendete Lösungsmittelkomponente auch geeignet ist um andere Kühlwirkstoffe zu lösen.

Eine erfindungsgemäße Verwendung ist bevorzugt, wobei es sich bei dem Kühlwirkstoff, bei zumindest einem der mehreren Kühlwirkstoffe oder bei sämtlichen der mehreren Kühlwirksotff um Kühlwirkstoffe handelt ausgewählt aus der Gruppe umfassend (1R,2S,5R)-N-(4-Methoxyphenyl)-5-methyl-2-(1-methylethyl)-cyclohexan-carboxamid (FEMA GRAS 4681), Menthol Methyl Ether (FEMA GRAS 4054), Menthoxyethanol (FEMA GRAS 4154), Menthone Glyceryl Acetal (FEMA GRAS 3807 und 3808), Mentholethylenglycolcarbonat (FEMA GRAS 3805), Menthyl-N-ethyloxamat, Monomethylsuccinat (FEMA GRAS 3810), Monomenthylglutarat (FEMA GRAS 4006), Menthoxy-1,2-propanediol (FEMA GRAS 3784),-Menthoxy-2-methyl-1,2-propandiol (FEMA GRAS 3849), Isopropyltrimethylbutyramid (WS-23, FEMA GRAS 3804), N-((Ethoxycarbonyl)methyl)-p-menthyl-3-carboxamid (WS-5, FEMA GRAS 4309), Diethylhydroxydimethylethylbutanamid (WS-116, FEMA GRAS 4603), Ethyldiisopropylbutanamid (WS-27, FEMA GRAS 4557), Cyclopropy-Imenthylcarboxamid (FEMA GRAS 4693), Mentholethylenglycolcarbonat (FEMA GRAS 3805), Menthanediol (FEMA GRAS 4053), Menthylpyrrolidoncarboxylat (FEMA GRAS 4155), Dimethylmenthylsuccinamid (FEMA GRAS 4230), 3,9-Dimethyl-6-(1-methylethyl)-1,4-dioxaspiro[4.5]decan-2-on (FEMA GRAS 4285), Menthylhydroxybutyrat (FEMA GRAS 4308), Menthyl acetoacetate (FEMA GRAS 4327), N-(4-cyanomethylphenyl)-p-menthyl-carboxamid (FEMA GRAS 4496), N-(2-pyridin-2-ylethyl)menthylcarboxamid (FEMA GRAS 4549), N-Hydroxyethyldimethyl-isopropylbutanamid (FEMA GRAS 4602), Dimenthylglutarat (FEMA GRAS 4604), N-[4-(2-Amino-2-oxoethyl)phenyl]-p-menthylcarboxamid (FEMA GRAS 4684), Menthoxyethoxyethanol (FEMA GRAS 4718), Hydroxymethylcyclohexylethanon (FEMA GRAS 4742), 2-(4-Methylphenoxy)-N-(1H-pyrazol-3-yl)-N-(thiophen-2-ylmethyl)acetamid (FEMA GRAS 4809), 2-(4-Ethylphenoxy)-*N*-(1H-pyrazol-3-yl)-*N*-(thiophen-2-ylmethyl)acetamid (FEMA GRAS 4880), *N*-(3-Hydroxy-4-methoxyphenyl)-2-isopropyl-5,5-dimethylcyclohexancarboxamid (FEMA GRAS 4881), N-(4-(Cyanomethyl)phenyl)-2-isopropyl-5,5-dimethylcyclohexancarboxamid (FEMA GRAS 4882) und N-(2-Hydroxy-2-phenylethyl)-2-isopropyl-5,5-dimethylcyclohexan-1-carboxamid (FEMA GRAS 4896).

Im Rahmen der vorliegenden Erfindung werden vorzugsweise mehrere der vorstehend als bevorzugt bezeichneten Aspekte gleichzeitig verwirklicht; insbesondere bevorzugt sind die sich aus den beigefügten Ansprüchen ergebenden Kombinationen solcher Aspekte und der entsprechenden Merkmale.

### Beispiele:

### Beispiel 1:

96 g 2-Hydroxypropyl(2-isopropyl-5-methyl-cyclohexyl)carbonat wurden in einem 250 mL Becherglas vorgelegt und 4 g (1R,2S,5R)-*N*-(4-Methoxyphenyl)-5-methyl-2-(1-methyl-ethyl)-cyclohexan-carboxamid wurden hinzugegeben. Die Mischung wurde gerührt und in 20°C Schritten erwärmt bis eine klare Lösung gebildet wurde. Nach dem jeweiligen Erwärmen um 20°C wurde die Lösung für mindestens 20 Minuten gerührt. Im Anschluss wurde die Mischung auf eine Raumtemperatur von ca. 25°C abkühlen lassen. Die hergestellte Lösung war auch nach dem Abkühlen für 8 Stunden stabil und es bildeten sich keine Kristalle.

### Beispiel 2:

Beispiel 1 wurde wiederholt, allerdings wurde statt 2-Hydroxypropyl(2-isopropyl-5-methylcyclohexyl)carbonat (2-Isopropyl-5-methyl-cyclohexyl)2-hydroxypropanoat als Lösungsmittel verwendet. Die hergestellte Lösung war auch nach dem Abkühlen stabil und es bildeten sich keine Kristalle.

### Beispiel 3:

Beispiel 1 wurde wiederholt, allerdings wurde statt 2-Hydroxypropyl(2-isopropyl-5-methylcyclohexyl)carbonat eine Mischung aus 2-Hydroxypropyl(2-isopropyl-5-methyl-cyclohexyl)carbonat und (2-Isopropyl-5-methyl-cyclohexyl)2-hydroxypropanoat als Lösungsmittel verwendet. Die hergestellte Lösung war auch nach dem Abkühlen stabil und es bildeten sich keine Kristalle.

### Beispiel 4:

Beispiel 1 wurde wiederholt, allerdings wurde statt 2-Hydroxypropyl(2-isopropyl-5-methylcyclohexyl)carbonat eine Mischung aus 2-Hydroxypropyl(2-isopropyl-5-methyl-cyclohexyl)carbonat, (2-Isopropyl-5-methyl-cyclohexyl)2-hydroxypropanoat und N-Ethyl-2-isopropyl-5-methyl-cyclohexanecarboxamid als Lösungsmittel verwendet und es wurden 10 g (1R,2S,5R)-*N*-(4-Methoxyphenyl)-5-methyl-2-(1-methylethyl)-cyclohexan-carboxamid gelöst. Die hergestellte Lösung war auch nach dem Abkühlen stabil und es bildeten sich erst nach über 24 Stunden Kristalle.

### Beispiel 5:

Beispiel 4 wurde wiederholt, allerdings wurden 8 g (1R,2S,5R)-*N*-(4-Methoxyphenyl)-5-methyl-2-(1-methylethyl)-cyclohexan-carboxamid gelöst. Die hergestellte Lösung war auch nach dem Abkühlen für mehr als 24 Stunden stabil und es bildeten sich keine Kristalle.

### Beispiel 6:

Beispiel 4 wurde wiederholt, allerdings wurden 6 g (1R,2S,5R)-*N*-(4-Methoxyphenyl)-5-methyl-2-(1-methylethyl)-cyclohexan-carboxamid gelöst. Die hergestellte Lösung war auch nach dem Abkühlen für mehr als 24 Stunden stabil und es bildeten sich keine Kristalle.

### Beispiel 7:

Beispiel 4 wurde wiederholt, allerdings wurden 4 g (1R,2S,5R)-*N*-(4-Methoxyphenyl)-5-methyl-2-(1-methylethyl)-cyclohexan-carboxamid gelöst. Die hergestellte Lösung war auch nach dem Abkühlen für mehr als zwei Wochen stabil und es bildeten sich keine Kristalle.

### Beispiel 8:

Beispiel 1 wurde wiederholt, allerdings wurde statt 2-Hydroxypropyl(2-isopropyl-5-methylcyclohexyl)carbonat eine Mischung aus 2-Hydroxypropyl(2-isopropyl-5-methyl-cyclohexyl)carbonat, (2-Isopropyl-5-methyl-cyclohexyl)2-hydroxypropanoat, N-Ethyl-2-isopropyl-5-methyl-cyclohexanecarboxamid und 6 Gew.-% mittelkettige Triglyceride als Lösungsmittel verwendet und es wurden 6 g (1R,2S,5R)-*N*-(4-Methoxyphenyl)-5-methyl-2-(1-methyl-ethyl)-cyclohexan-carboxamid gelöst. Die hergestellte Lösung war auch nach dem Abkühlen für mehr als zwei Wochen stabil und es bildeten sich keine Kristalle.

### Beispiel 9: Herstellung von Granulatpartikeln mit einer erfindungsgemäßen kühlenden Zusammensetzung mittels Sprühgranulation:

Eine erfindungsgemäße kühlende Zusammensetzung aus Beispiel 8 wurde hergestellt und bei ca. 20°C gelagert. Anschließend wurde eine Mischung aus 50 Gewichtsteilen modifizierter Stärke, 25 Gewichtsteilen Gummi arabicum (Senegal) und 10 Gewichtsteilen Mannitol als Trägerkomponente bereitgestellt. Die kühlende Zusammensetzung und die Trägerkomponente wurden in einem Gewichtsverhältnis von 1 zu 4 gemischt und sprühgranuliert.

Man erhält kühlende Partikel mit einem mittels Laserlichtbeugung nach ISO ISO13320:2009-10 bestimmten mittleren Partikeldurchmesser D50 von 600 µm, die ca. 20 Gew.-% der erfindungsgemäßen kühlenden Zusammensetzung umfassen.

Durch Variation der Prozessparameter des Sprühgranulationsprozesses ist es auch möglich, Partikel mit einer Größe von beispielsweise 200µm, 300 µm, 400 µm, 800 µm, 1400 µm, 1800 µm oder 2000 µm herstellen.

### Beispiel 10: Herstellung von Partikeln mit einer erfindungsgemäßen kühlenden Zusammensetzung mittels Sprühtrocknung:

Eine erfindungsgemäße kühlende Zusammensetzung aus Beispiel 8 wurde hergestellt und bei ca. 20°C gelagert. Anschließend wurde eine Mischung aus 60 Gewichtsteilen modifizierter Stärke, 25 Gewichtsteilen Gummi arabicum (Senegal) und 10 Gewichtsteilen Zuckeralkohol als Trägerkomponente bereitgestellt. Die kühlende Zusammensetzung und die Trägerkomponente wurden in einem Gewichtsverhältnis von 3 zu 7 gemischt und sprühgetrocknet.

Man erhält kühlende Partikel mit einem mittels Laserlichtbeugung nach ISO ISO13320:2009-10 bestimmten mittleren Partikeldurchmesser D50 von 80 µm, die ca. 27 Gew.-% der erfindungsgemäßen kühlenden Zusammensetzung umfassen.

Durch Variation der Prozessparameter des Sprühtrocknungsprozesses ist es auch möglich, Partikel mit einer Größe von beispielsweise 10 µm, 20 µm, 75 µm, 125 µm, 150 µm, 175 µm oder 200 µm herstellen.

### Beispiel 11: Herstellung eines Mundwassers:

| **Teil** | **Inhaltsstoff** | **Einsatz in Gew.-%** |
|---|---|---|
| A | Ethanol | 10,00 |
| | Cremophor^{®} CO 40 (BASF, Detergenz) | 1,00 |
| | Benzoesäure | 0,12 |
| | Lösung aus Beispiel 8 | 1,00 |
| B | demineralisiertes Wasser | 82,71 |
| | Sorbitol, 70% | 5,00 |
| | Natriumsaccharin 450 | 0,07 |
| | L-Blue 5000 e.c., 1% in Wasser (Farbstoff) | 0,10 |

Die Inhaltsstoffe der Teile A und B wurden jeweils für sich gemischt. Teil B wurde langsam in Teil A eingerührt, bis die Mischung homogen war.

Bei der Verwendung des so hergestellten Mundwassers konnte eine sehr hohe Kühlwirkung festgestellt werden.

### Beispiel 12: Herstellung eines zuckerfreien Kaugummis:

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylitol | 2,00 |
| | Mannitol | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70% | 14,00 |
| | Glycerin | 1,00 |
| D | Granulat aus Beispiel 9 | 1,00 |

Teile A bis D wurden gemischt und intensiv geknetet. Die Rohmasse wurde z.B. in Form von dünnen Streifen zu verzehrsfertigen Kaugummis verarbeitet.

Bei der Verwendung des so hergestellten Kaugummis konnte eine deutliche Kühlwirkung festgestellt werden.

### Vergleichsbeispiel 1:

Beispiel 1 wurde wiederholt, allerdings wurde statt 2-Hydroxypropyl(2-isopropyl-5-methylcyclohexyl)carbonat Ethanol als Lösungsmittel verwendet. Die hergestellte Lösung kristallisierte bereits beim Abkühlen auf Raumtemperatur aus.

### Vergleichsbeispiel 2:

Beispiel 1 wurde wiederholt, allerdings wurden statt 2-Hydroxypropyl(2-isopropyl-5-methylcyclohexyl)carbonat mittelkettige Triglyceride als Lösungsmittel verwendet und es wurde 1 g (1R,2S,5R)-*N*-(4-Methoxyphenyl)-5-methyl-2-(1-methylethyl)-cyclohexan-carboxamid gelöst. Es ließ sich keine Lösung herstellen. (1R,2S,5R)-*N*-(4-Methoxyphenyl)-5-methyl-2-(1-methylethyl)-cyclohexan-carboxamid ist in mittelkettigen Triglyceriden auch bei 60°C unlöslich.

### Vergleichsbeispiel 3:

Beispiel 1 wurde wiederholt, allerdings wurde statt 2-Hydroxypropyl(2-isopropyl-5-methylcyclohexyl)carbonat N-Ethyl-2-isopropyl-5-methyl-cyclohexanecarboxamid als Lösungsmittel verwendet, es wurde 1 g (1R,2S,5R)-*N*-(4-Methoxyphenyl)-5-methyl-2-(1-methyl-ethyl)-cyclohexan-carboxamid gelöst und die Mischung wurde bis zur Schmelze beider Substanzen erhitzt. Beide Substanzen kristallisierten beim Abkühlen aus.

## Patentansprüche

1. Kühlende Zusammensetzung zur Herstellung von kühlenden Partikeln bestehend aus oder umfassend
(A) eine Lösungsmittelkomponente bestehend aus oder umfassend
i) 2-Hydroxypropyl (2-isopropyl-5-methyl-cyclohexyl) carbonat
und/oder
ii) (2-Isopropyl-5-methyl-cyclohexyl) 2-hydroxypropanoat
und darin gelöst
(B) 0,5 bis 12 Gew.-% (1R,2S,5R)-*N*-(4-Methoxyphenyl)-5-methyl-2-(1-methyl-ethyl)-cyclohexan-carboxamid (CAS Nummer 68489-09-8; FEMA Nummer 4681) als Kühlwirkstoff, bezogen auf das Gesamtgewicht der Zusammensetzung.

2. Zusammensetzung nach Anspruch 1, wobei die Zusammensetzung
A) 0,5 bis 6 Gew.-%, vorzugsweise 1 bis 4 Gew.-%, besonders bevorzugt 1,5 bis 3 Gew.-% (1R,2S,5R)-N-(4-methoxyphenyl)-5-methyl-2-(1-methylethyl)-cyclohexane-carboxamid (FEMA 4681) als Kühlwirkstoff umfasst
oder
B) 6 bis 12 Gew.-%, vorzugsweise 6,5 bis 10 Gew.-%, besonders bevorzugt 7 bis 9 Gew.-% (1R,2S,5R)-*N*-(4-methoxyphenyl)-5-methyl-2-(1-methylethyl)-cyclohexane-carboxamid (FEMA 4681) als Kühlwirkstoff umfasst,
jeweils bezogen auf das Gesamtgewicht der Zusammensetzung.

3. Zusammensetzung nach einem der Ansprüche 1 oder 2, wobei die Zusammensetzung zusätzlich N-Ethyl-2-isopropyl-5-methyl-cyclohexanecarboxamid enthält.

4. Zusammensetzung nach einem der vorherigen Ansprüche,
wobei es sich bei 2-Hydroxypropyl (2-isopropyl-5-methyl-cyclohexyl) carbonat um 2-Hydroxypropyl [(1R,2S,5R)-2-isopropyl-5-methyl-cyclohexyl] carbonat (CAS Nummer 260781-16-6) handelt,
und/oder
wobei es sich bei N-Ethyl-2-isopropyl-5-methyl-cyclohexanecarboxamid um (1R,2S,5R)-*N*-Ethyl-2-isopropyl-5-methyl-cyclohexanecarboxamid (CAS Nummer 68489-00-9) handelt,
und/oder
wobei es sich bei (2-Isopropyl-5-methyl-cyclohexyl) 2-hydroxypropanoat um [(1R,2S,5R)-2-Isopropyl-5-methyl-cyclohexyl] (2S)-2-hydroxypropanoat (CAS Nummer 61597-98-6) handelt.

5. Zusammensetzung nach einem der vorherigen Ansprüche,
wobei die Lösungsmittelkomponente 2 bis 20 Gew.-%, vorzugsweise 4 bis 15 Gew.-%, besonders bevorzugt 6,4 bis 11,8 Gew.-% 2-Hydroxypropyl (2-isopropyl-5-methyl-cyclohexyl) carbonat umfasst, bezogen auf das Gesamtgewicht der Lösungsmittelkomponente
und/oder
wobei die Lösungsmittelkomponente 15 bis 35 Gew.-%, vorzugsweise 20 bis 30 Gew.-%, besonders bevorzugt 23 bis 27 Gew.-% N-Ethyl-2-isopropyl-5-methyl-cyclohexanecarboxamid umfasst, bezogen auf das Gesamtgewicht der Lösungsmittelkomponente.

6. Zusammensetzung nach einem der vorherigen Ansprüche, wobei die Lösungsmittelkomponente 55 bis 75 Gew.-%, vorzugsweise 60 bis 70 Gew.-%, besonders bevorzugt 63 bis 67 Gew.-% (2-Isopropyl-5-methyl-cyclohexyl) 2-hydroxypropanoat umfasst, bezogen auf das Gesamtgewicht der Lösungsmittelkomponente.

7. Zusammensetzung nach einem der vorherigen Ansprüche, wobei die Zusammensetzung 88 bis 99,5 Gew.-%, vorzugsweise 90 bis 94 Gew.-% der Lösungsmittelkomponente (A) enthält, bezogen auf das Gesamtgewicht der Zusammensetzung.

8. Zusammensetzung nach einem der Ansprüche 1 bis 5, wobei die Zusammensetzung zusätzlich 0,5 bis 8 Gew.-%, vorzugsweise 4 bis 7,5 Gew.-%, besonders bevorzugt 5 bis 7 Gew.-% mittelkettige Triglyceride enthält, bezogen auf das Gesamtgewicht der Zusammensetzung,
vorzugsweise wobei die Zusammensetzung 80 bis 99 Gew.-%, vorzugsweise 80,5 bis 95,5 Gew.-%, besonders bevorzugt 81 bis 94,5 Gew.-% der Lösungsmittelkomponente (A) enthält, bezogen auf das Gesamtgewicht der Zusammensetzung.

9. Zusammensetzung nach einem der vorherigen Ansprüche, wobei die Zusammensetzung mehr als 50 Gew.-%, vorzugsweise mehr als 65 Gew.-%, besonders bevorzugt mehr als 80 Gew.-% der Lösungsmittelkomponente (A) enthält, bezogen auf das Gesamtgewicht der Zusammensetzung.

10. Kühlende Partikel umfassend eine Zusammensetzung nach einem der Ansprüche 1 bis 9, vorzugsweise wobei die Partikel einen mittels Laserlichtbeugung nach ISO ISO13320:2009-10 bestimmten mittleren Partikeldurchmesser D50 im Bereich von 10 bis 2000 µm aufweisen.

11. Orale Zubereitung, umfassend kühlende Partikel nach Anspruch 10 oder eine Zusammensetzung nach einem der Ansprüche 1 bis 9,
vorzugsweise wobei die orale Zubereitung ein Getränk, ein Kaugummi, ein Mundwasser, Zahnpasta, Lippenbalsam oder ein Bonbon ist.

12. Orale Zubereitung nach Anspruch 11, umfassend a) 5 bis 95 Gew.-% Kaugummibasis, b) 5 bis 95 Gew.-% Füll- und Süßungsmittel, c) 0,1 bis 15 Gew.-% Geschmackstoffe, d) 0,2 bis 4 Gew.-% kühlende Partikel nach Anspruch 10.

13. Verwendung von kühlenden Partikeln nach Anspruch 10 oder einer Zusammensetzung nach einem der Ansprüche 1 bis 9, um in oralen Zubereitungen ein Frischegefühl durch eine physiologisch kühlende Wirkung zu vermitteln.

14. Verwendung einer Zusammensetzung nach einem der Ansprüche 1 bis 9 in einem Verkapselungsverfahren, vorzugsweise einer Sprühtrocknung oder Sprühgranulation.

15. Verfahren zur Herstellung von kühlenden Partikeln umfassend die folgenden Schritte:
- Herstellen oder Bereitstellen einer Zusammensetzung nach einem der Ansprüche 1 bis 9,
- Lagern der hergestellten oder bereitgestellten Zusammensetzung,
- Herstellen oder Bereitstellen von Matrixmaterialien,
- gegebenenfalls Mischen der hergestellten oder bereitgestellten Zusammensetzung mit den hergestellten oder bereitgestellten Matrixmaterialien,
- Durchführen eines Verkapselungsverfahrens, sodass kühlende Partikel resultieren, in denen die Zusammensetzung von den Matrixmaterialien umschlossen wird.

16. Verfahren nach Anspruch 15,
wobei es sich bei dem Verkapselungsverfahren um Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren handelt, vorzugsweise um Sprühtrocknung oder Sprühgranulation handelt,
und/oder
wobei es sich bei den Matrixmaterialien um Stärke, Stärkederivate (z.B. modifizierte Stärke), Cellulose oder Cellulosederivate (z.B. Hydroxypropylcellulose), anderen Polysaccharide (z.B. Pektin, Dextrin, Alginat, Curdlan, Carageenan, Chitin, Chitosan, Pullulan, Gummi arabicum), natürliche Fette, natürliche Wachse (z.B. Bienenwachs, Carnaubawachs), Proteine, z.B. Gelatine und/oder sonstigen Naturprodukte (z.B. Schellack) handelt,
und/oder
wobei das Lagern der hergestellten oder bereitgestellten Zusammensetzung bei einer Temperatur zwischen 15°C und 65°C, vorzugsweise zwischen 18°C und 35°C, besonders bevorzugt zwischen 20 und 25°C erfolgt,
und/oder
wobei das Lagern der hergestellten oder bereitgestellten Zusammensetzung ohne Zuführung von Wärmeenergie erfolgt,
und/oder
wobei die Partikel einen mittels Laserlichtbeugung nach ISO ISO13320:2009-10 bestimmten mittleren Partikeldurchmesser D50 im Bereich von 10 bis 2000 µm aufweisen.

17. Verwendung einer Lösungsmittelkomponente bestehend aus oder umfassend
i) 2-Hydroxypropyl (2-isopropyl-5-methyl-cyclohexyl) carbonat
und/oder
ii) (2-Isopropyl-5-methyl-cyclohexyl) 2-hydroxypropanoat
zum Lösen von Kühlwirkstoffen,
wobei es sich bei dem Kühlwirkstoff, bei zumindest einem der mehreren Kühlwirkstoffe oder bei sämtlichen der mehreren Kühlwirkstoffe um Kühlwirkstoffe handelt ausgewählt aus der Gruppe umfassend (1R,2S,5R)-*N*-(4-Methoxyphenyl)-5-methyl-2-(1-methylethyl)-cyclohexan-carboxamid (FEMA GRAS 4681), Menthol Methyl Ether (FEMA GRAS 4054), Menthoxyethanol (FEMA GRAS 4154), Menthone Glyceryl Acetal (FEMA GRAS 3807 und 3808), Mentholethylenglycolcarbonat (FEMA GRAS 3805), Menthyl-N-ethyloxamat, Monomethylsuccinat (FEMA GRAS 3810), Monomenthylglutarat (FEMA GRAS 4006), Menthoxy-1,2-propanediol (FEMA GRAS 3784),-Menthoxy-2-methyl-1,2-propandiol (FEMA GRAS 3849), Isopropyltrimethylbutyramid (WS-23, FEMA GRAS 3804), N-((Ethoxycarbonyl)methyl)-p-menthyl-3-carboxamid (WS-5, FEMA GRAS 4309), Diethylhydroxydimethylethylbutanamid (WS-116, FEMA GRAS 4603), Ethyldiisopropylbutanamid (WS-27, FEMA GRAS 4557), Cyclopropylmenthylcarboxamid (FEMA GRAS 4693), Mentholethylenglycolcarbonat (FEMA GRAS 3805), Menthanediol (FEMA GRAS 4053), Menthylpyrrolidoncarboxylat (FEMA GRAS 4155), Dimethylmenthylsuccinamid (FEMA GRAS 4230), 3,9-Dimethyl-6-(1-methylethyl)-1,4-dioxaspiro[4.5]decan-2-on (FEMA GRAS 4285), Menthylhydroxybutyrat (FEMA GRAS 4308), Menthyl acetoacetate (FEMA GRAS 4327), N-(4-cyanomethylphenyl)-p-menthylcarboxamid (FEMA GRAS 4496), N-(2-pyridin-2-ylethyl)menthylcarboxamid (FEMA GRAS 4549), N-Hydroxyethyldimethyl-isopropylbutanamid (FEMA GRAS 4602), Dimenthylglutarat (FEMA GRAS 4604), N-[4-(2-Amino-2-oxoethyl)phenyl]-p-menthyl-carboxamid (FEMA GRAS 4684), Menthoxyethoxyethanol (FEMA GRAS 4718), Hydroxymethylcyclohexylethanon (FEMA GRAS 4742), 2-(4-Methylphenoxy)-N-(1H-pyrazol-3-yl)-N-(thiophen-2-ylmethyl)acetamid (FEMA GRAS 4809), 2-(4-Ethylphenoxy)-*N*-(1 H-pyrazol-3-yl)-N-(thiophen-2-ylmethyl)acetamid (FEMA GRAS 4880), N-(3-Hydroxy-4-methoxyphenyl)-2-isopropyl-5,5-dimethylcyclohexancarboxamid (FEMA GRAS 4881), *N*-(4-(Cyanomethyl)phenyl)-2-isopropyl-5,5-dimethylcyclohexancarboxamid (FEMA GRAS 4882) und N-(2-Hydroxy-2-phenylethyl)-2-isopropyl-5,5-dimethylcyclohexan-1-carboxamid (FEMA GRAS 4896).

## Claims

1. A cooling composition for producing cooling particles consisting of or comprising
(A) a solvent component consisting of or comprising
i) 2-hydroxypropyl (2-isopropyl-5-methyl-cyclohexyl) carbonate and/or
ii) (2-isopropyl-5-methyl-cyclohexyl) 2-hydroxypropanoate
and dissolved therein
(B) 0.5 to 12 wt.-% of (1R,2S,5R)-N-(4-methoxyphenyl)-5-methyl-2-(1-methylethyl)-cyclohexane carboxamide (CAS number 68489-09-8; FEMA number 4681), based on the total weight of the composition, as cooling agent.

2. The composition according to claim 1, wherein the composition
A) comprises 0.5 to 6 wt.-%, preferably 1 to 4 wt.-%, more preferably 1.5 to 3 wt.-%, of (1R,2S,5R)-N-(4-methoxyphenyl)-5-methyl-2-(1-methylethyl)-cyclohexanecarboxamide (FEMA 4681) as cooling agent
or
B) comprises 6 to 12 wt.-%, preferably 6.5 to 10 wt.-%, more preferably 7 to 9 wt.-% of (1R,2S,5R)-N-(4-methoxyphenyl)-5-methyl-2-(1-methylethyl)-cyclohexanecarboxamide (FEMA 4681) as cooling agent
based on the total weight of the composition, respectively.

3. The composition according to claim 1 or 2, wherein the composition additionally comprises N-ethyl-2-isopropyl-5-methyl-cyclohexane carboxamide.

4. The composition according to any of the preceding claims,
wherein 2-hydroxypropyl (2-isopropyl-5-methyl-cyclohexyl) carbonate is 2-hydroxypropyl [(1R,2S,5R)-2-isopropyl-5-methyl-cyclohexyl] carbonate (CAS number 260781-16-6),
and/or
wherein N-ethyl-2-isopropyl-5-methyl-cyclohexane carboxamide is (1R,2S,5R)-N-ethyl-2-isopropyl-5-methyl-cyclohexane carboxamide (CAS number 68489-00-9),
and/or
wherein (2-isopropyl-5-methyl-cyclohexyl) 2-hydroxypropanoate is [(1R,2S,5R)-2-isopropyl-5-methyl-cyclohexyl]-(2S)-2-hydroxypropanoate (CAS number 61597-98-6).

5. The composition according to any of the preceding claims,
wherein the solvent component comprises 2 to 20 wt.-%, preferably 4 to 15 wt.-%, more preferably 6.4 to 11.8 wt.-%, based on the total weight of the solvent component, of 2-hydroxypropyl (2-isopropyl-5-methyl-cyclohexyl) carbonate
and/or
wherein the solvent component comprises 15 to 35 wt.-%, preferably 20 to 30 wt.-%, more preferably 23 to 27 wt.-%, based on the total weight of the solvent component, of N-ethyl-2-isopropyl-5-methyl-cyclohexane carboxamide.

6. The composition according to any of the preceding claims, wherein the solvent component comprises 55 to 75 wt.-%, preferably 60 to 70 wt.-%, more preferably 63 to 67 wt.-%, based on the total weight of the solvent component, of (2-isopropyl-5-methyl-cyclohexyl) 2-hydroxypropanoate.

7. The composition according to any of the preceding claims, wherein the composition comprises 88 to 99.5 wt.-%, preferably 90 to 94 wt.-% ,based on the total weight of the composition, of the solvent component (A).

8. The composition according to any of claims 1 to 5, wherein the composition additionally comprises 0.5 to 8 wt.-%, preferably 4 to 7.5 wt.-%, more preferably 5 to 7 wt.-%, based on the total weight of the composition, of medium-chain triglycerides, preferably wherein the composition comprises 80 to 99 wt.-%, preferably 80.5 to 95.5 wt.-%, more preferably 81 to 94.5 wt.-%, based on the total weight of the composition, of the solvent component (A).

9. The composition according to any of the preceding claims, wherein the composition comprises more than 50 wt.-%, preferably more than 65 wt.-%, more preferably more than 80 wt.-%, based on the total weight of the composition, of the solvent component (A).

10. Cooling particles comprising a composition according to any of claims 1 to 9, preferably wherein the particles have a mean particle diameter D50 in the range of 10 µm to 2000 µm as determined by laser light diffraction according to ISO ISO13320:2009-10.

11. An oral preparation, comprising the cooling particles according to claim 10 or a composition according to any of claims 1 to 9,
preferably wherein the oral preparation is a beverage, a chewing gum, a mouthwash, a toothpaste, a lip balm or a candy.

12. The oral preparation according to claim 11, comprising a) 5 to 95 wt.-% of a chewing gum base, b) 5 to 95 wt.-% of filling agents and sweeteners, c) 0.1 to 15 wt.-% of flavourings, d) 0.2 to 4 wt.-% of cooling particles according to claim 10.

13. Use of cooling particles according to claim 10 or a composition according to any of claims 1 to 9, to impart a feeling of freshness in oral preparations through a physiologically cooling effect.

14. Use of a composition according to any of claims 1 to 9, in an encapsulation process, preferably spray-drying or spray granulation.

15. A process for preparing cooling particles comprising the following steps:
- Producing or providing a composition according to any of claims 1 to 9,
- storing the produced or provided composition,
- producing or providing matrix materials,
- optionally, mixing the produced or provided composition with the produced or provided matrix materials,
- carrying out an encapsulation process such that cooling particles are obtained, in which the composition is enclosed by the matrix material.

16. The process according to claim 15,
wherein the encapsulation process is spray-drying, spray granulation, melt granulation, coacervation, coagulation, extrusion, melt extrusion, emulsion process, coating or other suitable encapsulation processes, preferably is spray-drying or spray granulation,
and/or
wherein the matrix material is starch, starch derivatives (e.g., modified starch), cellulose or cellulose derivatives (e.g., hydroxypropyl cellulose), other polysaccharides (e.g., pectin, dextrin, alginate, curdlan, carrageenan, chitin, chitosan, pullulan, gum arabic), natural fats, natural waxes (e.g. beeswax, carnauba wax), proteins, e.g., gelatin and/or other natural products (e.g., shellac),
and/or
wherein the storing of the produced or provided composition is performed at a temperature between 15°C and 65°C, preferably between 18°C and 35°C, more preferably between 20°C and 25°C,
and/or
wherein the storing of the produced or provided composition is performed without applying heat energy,
and/or
wherein the particles have a mean particle diameter D50 in the range of 10 to 2000 µm as determined by laser light diffraction according to ISO ISO13320:2009-10.

17. Use of a solvent component consisting of or comprising
i) 2-hydroxypropyl (2-isopropyl-5-methylcyclohexyl) carbonate
and/or
ii) (2-isopropyl-5-methyl-cyclohexyl) 2-hydroxypropanoate
to dissolve cooling agents,
wherein the cooling agent, at least one of the multiple cooling agents, or all of the multiple cooling agents is/are cooling agents selected from the group comprising (1R,2S,5R)-N-(4-methoxyphenyl)-5-methyl-2-(1-methylethyl)-cyclohexanecarboxamide (FEMA GRAS 4681), menthol methyl ether (FEMA GRAS 4054), menthoxyethanol (FEMA GRAS 4154), menthone glyceryl acetal (FEMA GRAS 3807 and 3808), menthol ethylenglycolcarbonate (FEMA GRAS 3805), menthyl-N-ethylox-amate, monomethyl succinate (FEMA GRAS 3810), monomenthyl glutarate (FEMA GRAS 4006), menthoxy-1,2-propane diol (FEMA GRAS 3784), -menthoxy-2-methyl-1,2-propane diol (FEMA GRAS 3849), isopropyl trimethyl butyramide (WS-23, FEMA GRAS 3804), N-((ethoxycarbonyl)methyl)-p-menthane-3-carboxamide (WS-5, FEMA GRAS 4309), diethylhydroxydimethylethylbutanamide (WS-116, FEMA GRAS 4603), ethyldiisopropylbutanamide (WS-27, FEMA GRAS 4557), cyclopropylmenthylcarboxamide (FEMA GRAS 4693), mentholethylen glycol carbonate (FEMA GRAS 3805), menthanediol (FEMA GRAS 4053), menthyl pyrrolidone carboxylate (FEMA GRAS 4155), dimethyl menthyl succinamide (FEMA GRAS 4230), 3,9-dimethyl-6-(1-methylethyl)-1,4-dioxaspiro[4.5]decan-2-one (FEMA GRAS 4285), menthyl hydroxybutyrate (FEMA GRAS 4308), menthyl acetoacetate (FEMA GRAS 4327), N-(4-cyanomethylphenyl)-p-menthylcarboxamide (FEMA GRAS 4496), N-(2-(pyridin-2-yl)ethyl)menthylcarboxamide (FEMA GRAS 4549), N-hydroxyethyldimethylisopropylbutanamide (FEMA GRAS 4602), dimenthyl glutarate (FEMA GRAS 4604), N-[4-(2-amino-2-oxoethyl)phenyl]-p-menthylcarboxamide, (FEMA GRAS 4684), menthoxyethoxyethanol (FEMA GRAS 4718), hydroxymethylcyclohexylethanone (FEMA GRAS 4742), 2-(4-Methylphenoxy)-N-(1H-pyrazol-3-yl)-N-(thiophen-2-ylmethyl)acetamide (FEMA GRAS 4809), 2-(4-Ethylphenoxy)-N-(1H-pyrazol-3-yl)-N-(thiophen-2-ylmethyl)acetamide (FEMA GRAS 4880), N-(3-Hydroxy-4-methoxyphenyl)-2-isopropyl-5,5-dimethylcyclohexanecarboxamide (FEMA GRAS 4881), N-(4-(cyanomethyl)phenyl)-2-isopropyl-5,5-dimethylcyclohexanecarboxamide (FEMA GRAS 4882) and N-(2-hydroxy-2-phenylethyl)-2-isopropyl-5,5-dimethylcyclohexane-1-carboxamide (FEMA GRAS 4896).

## Revendications

1. Composition refroidissante pour la production de particules refroidissantes constituées ou comprenant
(A) un composant solvant constitué ou comprenant
i) du carbonate de 2-hydroxypropyle (2-isopropyl-5-méthylcyclohexyle)
et/ou
ii) du 2-hydroxypropanoate de (2-isopropyl-5-méthylcyclohexyle)
et dissous dans celui-ci
(B) 0,5 à 12 % en poids de (1R,2S,5R)-N-(4-méthoxyphényl)-5-méthyl-2-(1-méthyléthyl)cyclohexane-carboxamide (numéro CAS 68489-09-8 ; numéro FEMA 4681) comme agent de refroidissement, par rapport au poids total de la composition.

2. Composition selon la revendication 1, dans laquelle la composition comprend
A) 0,5 à 6 % en poids, de préférence 1 à 4 % en poids, de manière particulièrement préférée 1,5 à 3 % en poids de (1R,2S,5R)-N-(4-méthoxyphényl)-5-méthyl-2-(1-méthyléthyl)-cyclohexane-carboxamide (FEMA 4681) comme agent de refroidissement
ou
B) 6 à 12 % en poids, de préférence 6,5 à 10 % en poids, de manière particulièrement préférée 7 à 9 % en poids de (1R,2S,5R)-N-(4-méthoxyphényl)-5-méthyl-2-(1-méthyléthyl)-cyclohexane-carboxamide (FEMA 4681) comme agent de refroidissement,
respectivement par rapport au poids total de la composition.

3. Composition selon l'une quelconque des revendications 1 ou 2, dans laquelle la composition contient en outre du N-éthyl-2-isopropyl-5-méthyle-cyclohexane-carboxamide.

4. Composition selon l'une quelconque des revendications précédentes,
dans laquelle le carbonate de 2-hydroxypropyl (2-isopropyl-5-méthyle-cyclohexyle) est le carbonate de 2-hydroxypropyl [(1R,2S,5R)-2-isopropyl-5-méthylcyclohexyle] (numéro CAS 260781-16-6),
et/ou
dans laquelle le N-éthyl-2-isopropyl-5-méthyle-cyclohexanecarboxamide est le (1R,2S,5R)-N-éthyl-2-isopropyl-5-méthyle-cyclohexanecarboxamide (numéro CAS 68489-00-9),
et/ou
dans laquelle le 2-hydroxypropanoate de (2-isopropyl-5-méthylecyclohexyle) est le [(1R,2S,5R)-2-isopropyl-5-méthyle-cyclohexyl] (2S)-2-hydroxypropanoate (numéro CAS 61597-98-6).

5. Composition selon l'une quelconque des revendications précédentes,
dans laquelle le composant solvant comprend 2 à 20 % en poids, de préférence 4 à 15 % en poids, de manière particulièrement préférée 6,4 à 11,8 % en poids de carbonate de 2-hydroxypropyle (2-isopropyl-5-méthylcyclohexyle), par rapport au poids total du composant solvant
et/ou
dans laquelle le composant solvant comprend 15 à 35 % en poids, de préférence 20 à 30 % en poids, de manière particulièrement préférée 23 à 27 % en poids de *N*-éthyl-2-isopropyl-5-méthylcyclohexanecarboxamide, par rapport au poids total du composant solvant.

6. Composition selon l'une quelconque des revendications précédentes, dans laquelle le composant solvant comprend 55 à 75 % en poids, de préférence 60 à 70 %, de manière particulièrement préférée 63 à 67 % en poids de 2-hydroxypropanoate de (2-isopropyl-5-méthylcyclohexyle), par rapport au poids total du composant solvant.

7. Composition selon l'une quelconque des revendications précédentes, dans laquelle la composition contient 88 à 99,5 %, de préférence 90 à 94 % en poids du composant solvant (A), par rapport au poids total de la composition.

8. Composition selon l'une quelconque des revendications 1 à 5, dans laquelle la composition contient en outre 0,5 à 8 % en poids, de préférence 4 à 7,5 % en poids, de manière particulièrement préférée 5 à 7 % en poids, de triglycérides à chaîne moyenne, par rapport au poids total de la composition,
de préférence, dans laquelle la composition contient 80 à 99 % en poids, de préférence 80,5 à 95,5 % en poids, de manière particulièrement préférée 81 à 94,5 % en poids du composant solvant (A), par rapport au poids total de la composition.

9. Composition selon l'une quelconque des revendications précédentes, dans laquelle la composition contient plus de 50 % en poids, de préférence plus de 65 % en poids, de manière particulièrement préférée plus de 80 % en poids du composant solvant (A), par rapport au poids total de la composition.

10. Particules refroidissantes comprenant une composition selon l'une quelconque des revendications 1 à 9, de préférence dans lesquelles les particules présentent un diamètre moyen de particule D50, déterminé par diffraction de lumière laser selon la norme ISO ISO 13320:2009-10, qui est compris entre 10 et 2000 µm.

11. Préparation orale comprenant des particules refroidissantes selon la revendication 10 ou une composition selon l'une quelconque des revendications 1 à 9,
de préférence dans laquelle la préparation orale est une boisson, un chewing-gum, un bain de bouche, un dentifrice, un baume à lèvres ou un bonbon.

12. Préparation orale selon la revendication 11, comprenant a) 5 à 95 % en poids de base de chewing-gum, b) 5 à 95 % en poids de charges et d'édulcorants, c) 0,1 à 15 % en poids d'agents aromatisants, d) 0,2 à 4 % en poids de particules refroidissantes selon la revendication 10.

13. Utilisation de particules refroidissantes selon la revendication 10 ou d'une composition selon l'une quelconque des revendications 1 à 9 afin de conférer une sensation de fraîcheur dans des préparations orales par un effet physiologiquement refroidissant.

14. Utilisation d'une composition selon l'une quelconque des revendications 1 à 9 dans un procédé d'encapsulation, de préférence un séchage par atomisation ou une granulation par atomisation.

15. Procédé de production de particules refroidissantes comprenant les étapes suivantes consistant à:
- produire ou fournir une composition selon l'une quelconque des revendications 1 à 9,
- stocker la composition produite ou fournie,
- produire ou fournir des matériaux de matrice,
- le cas échéant mélanger la composition produite ou fournie avec les matériaux de matrice produits ou fournis,
- mettre en œuvre un procédé d'encapsulation de sorte qu'il en résulte des particules refroidissantes dans lesquelles la composition est enfermée dans les matériaux de matrice.

16. Procédé selon la revendication 15,
dans lequel le procédé d'encapsulation est le séchage par atomisation, la granulation par atomisation, la granulation à l'état fondu, la coacervation, la coagulation, l'extrusion, l'extrusion à l'état fondu, le procédé d'émulsion, l'enrobage (coating) ou d'autres procédés d'encapsulation appropriés, de préférence le séchage par atomisation ou la granulation par atomisation,
et/ou
dans lequel les matériaux de matrice sont l'amidon, des dérivés de l'amidon (par exemple l'amidon modifié), la cellulose ou des dérivés de la cellulose (par exemple, l'hydroxypropylcellulose), d'autres polysaccharides (par exemple la pectine, la dextrine, l'alginate, le curdlane, la carraghénane, la chitine, le chitosane, le pullulane, la gomme arabique), des graisses naturelles, des cires naturelles (par exemple la cire d'abeille, la cire de carnauba), des protéines, par exemple la gélatine, et/ou d'autres produits naturels (par exemple la gomme laque),
et/ou
dans lequel le stockage de la composition préparée ou fournie s'effectue à une température comprise entre 15 °C et 65 °C, de préférence entre 18 °C et 35 °C, de manière particulièrement préférée entre 20 et 25 °C,
et/ou
dans lequel le stockage de la composition préparée ou fournie s'effectue sans apport d'énergie thermique,
et/ou
dans lequel les particules présentent un diamètre moyen de particule D50, déterminé par diffraction de lumière laser selon la norme ISO ISO 13320:2009-10, qui est compris entre 10 et 2000 µm.

17. Utilisation d'un composant solvant constitué de ou comprenant
i) du carbonate de 2-hydroxypropyle (2-isopropyl-5-méthylcyclohexyle)
et/ou
ii) du 2-hydroxypropanoate de (2-isopropyl-5-méthylcyclohexyle)
pour dissoudre des agents de refroidissement,
dans lequel l'agent de refroidissement, au moins un agent parmi la pluralité d'agents de refroidissement, ou la totalité de la pluralité d'agents de refroidissement sont des agents de refroidissement choisis dans le groupe comprenant le (1R,2S,5R)-N-(4-méthoxyphényl)-5-méthyl-2-(1-méthyléthyl)cyclohexane-carboxamide (FEMA GRAS 4681), l'éther méthylique de menthol (FEMA GRAS 4054), le menthoxyéthanol (FEMA GRAS 4154) et l'acétal de glycéryle de menthone (FEMA GRAS 3807 et 3808), le carbonate d'éthylène glycol de menthol (FEMA GRAS 3805), oxamate de menthyl-N-éthyle, succinate de monométhyle (FEMA GRAS 3810), glutarate de monomenthyle (FEMA GRAS 4006), le menthoxy-1,2-propanediol (FEMA GRAS 3784), le menthoxy-2-méthyl-1,2-propanediol (FEMA GRAS 3849), isopropyltriméthylbutyramide (WS-23, FEMA GRAS 3804), N-((éthoxycarbonyl)méthyl)-p-menthyl-3-carboxamide (WS-5, FEMA GRAS 4309), diéthylhydroxydiméthyléthylbutanamide (WS-116, FEMA GRAS 4603), éthyldiisopropylbutanamide (WS-27, FEMA GRAS 4557), cyclopropylmenthylcarboxamide (FEMA GRAS 4693), carbonate de mentholéthylène glycol (FEMA GRAS 3805), menthanediol (FEMA GRAS 4053), carboxylate de menthylpyrrolidone (FEMA GRAS 4155), diméthylmenthylsuccinamide (FEMA GRASS 4230), 3,9-diméthyl-6-(1-méthyléthyl)-1,4-dioxaspiro[4.5]décan-2-one (FEMA GRAS 4285), menthylhydroxybutyrate (FEMA GRAS 4308), menthyle acétoacétate (FEMA GRAS 4327), N-(4-cyanométhylphényl)-p-menthylcarboxamide (FEMA GRAS 4496), N-(2-pyridin-2-yléthyl)menthylcarboxamide (FEMA GRAS 4549), N-hydroxyéthyldiméthyl-isopropylbutanamide (FEMA GRAS 4602), dimenthyle glutarate (FEMA GRAS 4604), N-[4-(2-amino-2-oxoéthyl)phényl]-p-menthylcarboxamide (FEMA GRAS 4684), menthoxyéthoxyéthanol (FEMA GRAS 4718), hydroxyméthylcyclohexyléthanone (FEMA GRAS 4742), 2-(4-méthylphénoxy)-N-(1H-pyrazol-3-yl)-N-(thiophén-2-ylméthyl)acétamide (FEMA GRAS 4809), 2-(4-éthylphénoxy)-*N*-(1H-pyrazol-3-yl)-*N*-(thiophén-2-ylméthyl)acétamide (FEMA GRAS 4880), *N*-(3-hydroxy-4-méthoxyphényl)-2-isopropyl-5,5-diméthylcyclohexanecarboxamide (FEMA GRAS 4881), *N*-(4-(cyanométhyl)phényl)-2-isopropyl-5,5-diméthylcyclohexanecarboxamide (FEMA GRAS 4882) et N-(2-hydroxy-2-phényléthyl)-2-isopropyl-5,5-diméthylcyclohexane-1-carboxamide (FEMA GRAS 4896).
